# FASCICULE DE BREVET EUROPEEN

(11) **EP 1 704 854 B1**
(45) Date de publication et mention de la délivrance du brevet: **22.02.2012**
(21) Numéro de dépôt: 06290130.1
(22) Date de dépôt: 19.01.2006
(51) Int. Cl.: A61K 8/90, A61Q 19/00, C08L 53/00

(54) **Dispersion de particules de polyméres**
Dispersion von Polymerteilchen
Dispersion of polymer particles

(30) Priorité: 04.02.2005 FR 0550340
(43) Date de publication de la demande: 27.09.2006
(73) Titulaire: L'Oréal, 75008 Paris (FR)
(72) Inventeur: Farcet, Céline, 75012 Paris (FR)
(74) Mandataire: Dodin, Catherine

(56) Documents cités:
- EP-A- 1 428 844
- EP-A1- 1 366 750
- WO-A-2004/055077
- WO-A2-2004/028487
- US-A- 5 219 560
- US-A- 5 711 940
- DATABASE CA CHEMICAL ABSTRACTS SERVICE, COLUMBUS, OHIO, US; XP002345515 extrait de STN Database accession no. 135: 293704 & JP 2001 278982 A (SHISEIDO CO., LTD) 10 octobre 2001 (2001-10-10)
- DATABASE CA [en ligne] CHEMICAL ABSTRACTS SERVICE, COLUMBUS, OHIO, US Database accession no. 141:319549 & JP 2004 277300 A (MITSUBISHI CHEM CORP) 7 October 2004 (2004-10-07)

## Description

La présente invention a trait à de nouvelles dispersions de particules de polymères, et à leur utilisation en cosmétique; l'invention concerne aussi les compositions notamment cosmétiques comprenant ces dispersions.

Il est connu d'utiliser en cosmétique des dispersions de particules de polymère, généralement de taille nanométrique, dans des milieux organiques.
Ainsi, dans la demande de brevet européen EP-A-0749747, il est décrit une composition cosmétique comprenant une dispersion de particules de polymère, dans un milieu non aqueux, ladite dispersion étant stabilisée par ajout de polymères stabilisants, qui se lient de manière non covalente par le biais d'interactions physiques sur les particules de polymère. Ce type de composition présente toutefois les inconvénients suivants : elle nécessite l'ajout dans le milieu non aqueux d'une quantité de polymères stabilisants supérieure à celle effectivement liée aux particules de polymères non solubles, afin d'obtenir une dispersion desdites particules relativement stable. Or, lors de l'ajout d'adjuvants tels que des pigments, dans les compositions, une partie des polymères stabilisants a tendance à se désorber des particules de polymères non solubles pour s'associer avec lesdits adjuvants, ce qui contribue à déstabiliser la dispersion, notamment par formation d'agglomérats entre les particules de polymères.
On connaît également, par la demande EP1428844, des compositions cosmétiques comprenant des dispersions, dans un milieu organique non aqueux non siliconé, de particules de polymères acryliques comprenant un squelette insoluble dans ledit milieu, et une partie soluble dans ledit milieu constituée de chaînes latérales liées de manière covalente audit squelette. Dans ce cas, les particules de polymères sont stabilisées par un polymère (macromère) qui est chimiquement lié aux particules de polymères.

Dans les deux cas, la nature du polymère stabilisant n'est pas très modulable, que cela soit en terme de nature chimique, de masse molaire et/ou d'architecture, et doit faire l'objet d'une synthèse spécifique. Par ailleurs, il n'est pas aisé de moduler les propriétés du coeur de la particule, que ce soit en terme de masse moléculaire et/ou d'architecture.

On connaît également par EP1428844 et WO2004/055077 des polymères greffés, en dispersion dans des huiles, se présentant sous forme de polymères ayant un squelette acrylique insoluble dans le milieu, et des greffons solubles dans ledit milieu. On connaît aussi par US5219560 des polymères greffés ayant un squelette acrylique et portant des greffons siliconés qui apportent la solubilité. Dans ces documents, il ne s'agit nullement de polymères séquencés linéaires, mais de polymères greffés.
On connaît enfin par le brevet JP2001278982 des polymères séquencés comprenant des blocs à greffons polysiloxanes et des blocs hydrophiles sulfoniques. Toutefois, il n'est pas précisé si le polymère est en dispersion dans le milieu considéré. Il est d'ailleurs fort probable qu'il soit en solution dans les huiles siliconées formant la majeure partie de la phase grasse de la composition cosmétique. Enfin WO2004/028487 décrit des dispersions cosmétiques de polyméres blocs dans un milieu organique.

La demanderesse a découvert de manière surprenante de nouvelles dispersions de particules de polymères, permettant d'obtenir de bonnes propriétés cosmétiques telles qu'une bonne adhésion sur le support (peau ou cheveu, notamment) et donc une bonne tenue de la composition cosmétique.
Par ailleurs, la dispersion selon l'invention ne comprenant pas de stabilisant au sens de l'art antérieur, elle est beaucoup plus stable, dans le temps, que les dispersions usuelles, ce qui implique une meilleure stabilité de la composition la comprenant et une facilité de formulation.
Enfin, le confort de la composition cosmétique est amélioré.

Un objet de la présente invention est une composition cosmétique comprenant, dans un milieu cosmétiquement acceptable, au moins une dispersion de particules de polymères dans un milieu carboné liquide, ledit polymère étant un copolymère comprenant au moins une première séquence soluble dans ledit milieu carboné et au moins une deuxième séquence insoluble dans ledit milieu carboné.

Grâce à l'invention, il est possible de moduler les propriétés physico-chimiques de la dispersion, et donc de la composition la comprenant, en choisissant de manière adéquate les monomères et le milieu organique la composant, possibilité qui n'était pas envisageable avec l'art antérieur. Ceci permet notamment d'obtenir une dispersion qui présente une grande affinité pour les milieux huileux, usuellement employés en cosmétique; ceci permet également de préparer une dispersion dont le dépôt ne présente pas de collant.

Les micelles de copolymère-bloc, notamment en milieu organique sont connues d'une manière générale. On peut notamment citer la demande brevet WO01/77198 qui est relative à un procédé de préparation de microgels par polymérisation RAFT en présence d'un agent de transfert de chaîne, consistant à préparer un copolymère-bloc comprenant des monomères solvophobes et des monomères solvophiles, puis à disperser ledit copolymère-bloc dans un milieu de dispersion pour former des micelles, qui seront stabilisées pour donner le microgel attendu. Dans ce document, le milieu de dispersion peut être organique, aqueux ou hydroorganique.
Toutefois, dans ce document, il n'est pas envisagé d'employer telles quelles les micelles ainsi préparées; elles servent à former des microgels par réticulation, lesdits microgels permettant d'encapsuler des pigments ou des colorants, par exemple, et étant susceptibles d'être employés dans divers domaines tels que le revêtement industriel.
Les microgels réticulés ainsi obtenus ne permettent pas d'obtenir des dépôts préférentiellement filmogènes, de bonne tenue et aisément démaquillables; or ceci constitue le principal objectif de la présente invention.

La dispersion de particules de polymères selon l'invention comprend donc un copolymère qui comprend au moins une première séquence soluble dans le milieu carboné et au moins une deuxième séquence insoluble dans ledit milieu carboné selon la présente revendication 1.

Par séquence, on entend dans la présente invention, un enchaînement polymérique, formé de plusieurs monomères, notamment d'au moins 5 monomères, identiques ou différents, et qui peut donc se présenter sous forme d'un homopolymère ou d'un copolymère statistique, alterné, à gradient ou bloc, notamment dibloc, tribloc ou multibloc.

De préférence, la séquence sera de type homopolymère ou à gradient.
Pour chaque séquence, le choix des monomères et de leur quantité, ainsi que de l'architecture de la séquence, pourra être effectué par l'homme du métier sur la base de ses connaissances générales de manière à obtenir au final une séquence ayant la solubilité requise (soluble ou insoluble) dans le milieu carboné considéré.

Au final, le copolymère obtenu est de préférence du type 'dibloc', c'est-à-dire qu'il comprend uniquement deux séquences, l'une étant soluble dans le milieu, l'autre y étant insoluble; il peut toutefois être du type 'tribloc', voire 'multiblocs' (plus de trois blocs).
De préférence, l'enchaînement des blocs solubles et insolubles est alterné. Chaque bloc ou séquence soluble peut être de longueur et/ou de masse molaire différente ou identique, de nature chimique différente ou identique, d'architecture différente ou identique. Chaque bloc ou séquence insoluble peut être de longueur ou masse molaire différente ou identique, de nature chimique différente ou identique, d'architecture différente ou identique.

De préférence, le copolymère selon l'invention est linéaire; il peut toutefois être ramifié et/ou greffé.
Le copolymère selon l'invention n'est pas réticulé; on entend par là qu'il n'y a pas d'ajout volontaire de composé ayant pour but de réticuler (agent réticulant).

Par soluble, on entend que la séquence est complètement dissoute (sans dépôt apparent, ni agglomérat ou sédiment insoluble), visuellement, à 20°C, à une concentration supérieure ou égale à 5% en poids, dans le milieu carboné considéré.

Les dispersions selon l'invention peuvent notamment se présenter sous forme de micelles polymériques (ou particules) en dispersion stable dans le milieu considéré. Ces micelles (ou particules) sont de préférence d'une taille comprise entre 5 et 1000 nm, de préférence 10 à 500 nm, encore mieux 20 à 300 nm, voire de 30 à 200 nm, ce qui permet d'obtenir une grande stabilité dans le temps de la dispersion.

Par micelles polymériques, on entend des particules autodispersées obtenues par auto-assemblage des copolymères tels que définis ci-après.
Ainsi, on peut considérer que la polymérisation du/des monomères composant la première séquence, d'amorceur et/ou d'agent de contrôle conduit à une première séquence soluble dans le milieu considéré. L'ajout du/des monomères destinés à composer le coeur de la particule aboutit à la formation du copolymère, généralement bloc, de type soluble/insoluble, copolymère qui va spontanément s'organiser en micelle polymérique, c'est-à-dire former une particule de polymère auto-dispersée en milieu carboné.

Un des avantages lié à la présente invention est qu'il est ainsi possible de former en une seule étape des particules de copolymère dispersées, dont les caractéristiques de la partie soluble et celles du coeur de la particule sont simultanément contrôlables.

Les copolymères selon la présente invention ont de préférence un poids moléculaire moyen en nombre (Mn) compris entre 1000 à 700 000, notamment entre 10000 et 500 000, et encore mieux entre 15000 et 350 000, voire entre 25 000 et 150 000.

De préférence, le copolymère selon l'invention présente un indice de polydispersité en masse (Ip) inférieur ou égal à 6, de préférence compris entre 1,05 et 4, notamment entre 1,1 et 3, voire entre 1,15 et 2,5.
L'indice de polydispersité en masse (Ip) du copolymère est égal au rapport de la masse moléculaire moyenne en poids (Mw) sur la masse moléculaire moyenne en nombre (Mn). Une faible polydispersité en masse traduit des longueurs de chaînes approximativement identiques.
On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique.

De préférence, la dispersion selon l'invention présente une polydispersité en taille de particules homogène, ce qui signifie que toutes les particules sont de la même taille; notamment, la dispersion est de préférence telle qu'au moins 50% en nombre des particules de la dispersion ont un diamètre identique ou quasi-identique (différence inférieure à 10%); ceci contribue à une meilleure stabilité de la dispersion dans la temps (pas de décantation, floculation et/ou sédimentation).
Ceci est un avantage par rapport à certaines dispersions de l'art antérieur, préparées selon les procédés de polymérisation radicalaire dits conventionnels; en effet, dans ces procédés, le produit obtenu est hétérogène en composition chimique car il s'agit généralement d'un mélange d'homopolymères et de copolymères.
Dans notre invention, la très grande majorité voire la totalité des chaînes (en fonction de la technique de polymérisation choisie) peut être sous forme de copolymère, ce qui aura pour particularité d'améliorer la stabilité des dispersions. Par ailleurs, les copolymères selon l'invention ont généralement des distributions en masse molaire et en compositions chimiques étroites et des masses molaires contrôlées ce qui permet de contrôler la taille des particules et leur distribution en taille.

Le copolymère selon l'invention comprend donc une première séquence soluble dans le milieu carboné de dispersion et au moins une seconde séquence, insoluble dans ledit milieu.

La séquence soluble comprend 50 à 100% en poids de monomère(s) soluble(s) dans ledit milieu, notamment de 60 à 90% en poids, et encore mieux de 70 à 80% en poids de monomère(s) soluble(s), seul ou en mélange. Elle comprend également 0 à 50% en poids, notamment de 10 à 40% en poids, voire de 20 à 30% en poids de monomère(s) insoluble(s) dans ledit milieu, seul ou en mélange.

De manière similaire, la séquence insoluble comprend 50 à 100% en poids de monomère(s) insoluble(s) dans ledit milieu, notamment de 60 à 90% en poids, et encore mieux de 70 à 80% en poids de monomère(s) insoluble(s), seul ou en mélange. Elle comprend également 0 à 50% en poids, notamment de 10 à 40% en poids, voire de 20 à 30% en poids de monomère(s) soluble(s) dans ledit milieu, seul ou en mélange.

L'homme du métier saura choisir, sur la base de ses connaissances générales, le ou les monomères solubles et insolubles, ainsi que leurs quantités, pour obtenir au final une séquence ayant la solubilité requise (soluble ou insoluble) dans le milieu carboné considéré.

Par monomère soluble dans le milieu selon la présente revendication1 on entend tout monomère dont l'homopolymère est sous forme soluble c'est-à-dire complètement dissous à une concentration supérieure ou égale à 5% en poids à température ambiante (20°C) dans ledit milieu.
Par monomère insoluble selon la presente revendication 1, on entend donc tout monomère dont l'homopolymère n'est pas sous forme soluble c'est-à-dire complètement dissous à une concentration supérieure ou égale à 5% en poids à température ambiante (20°C) dans ledit milieu. Toutefois, les monomères insolubles peuvent, en tant que monomères, être solubles dans le milieu considéré, étant entendu qu'ils deviennent insolubles après polymérisation.

En tout état de cause, la proportion de séquence soluble et de séquence insoluble dans le copolymère doit être telle que le copolymère puisse former une micelle polymérique.
De préférence, la séquence insoluble (ou les séquences insolubles), représente 30 à 97% en poids du poids total du copolymère, notamment de 40 à 95% en poids, voire de 50 à 93% en poids, notamment 60 à 92% en poids, et encore mieux de 75 à 90% en poids.
La séquence soluble (ou les séquences solubles), représente donc de préférence 3 à 70% en poids du poids total du copolymère, notamment de 5 à 60% en poids, voire de 7 à 50% en poids, notamment 8 à 40% en poids, et encore mieux de 10 à 25% en poids.

Comme monomère soluble susceptible d'être employé, on peut citer, seul ou en mélange, les monomères suivants :
- les méthacrylates de formule CH₂ = C(CH₃)-COOR₁
   dans laquelle R₁ représente un groupe alkyle linéaire ou ramifié en C8-C22 tel que lauryle, béhényle ou stéaryle; ou bien un groupe alkyle cyclique ayant 8 à 30 atomes de carbone, tel que isobornyle; ou encore R₁ représente le groupe tertiobutyle;
- les acrylates de formule CH₂ = CH-COOR₂
   dans laquelle R₂ représente un groupe alkyle linéaire ou ramifié en C8-C22 tel que lauryle, béhényle ou stéaryle ou 2-éthylhexyle; ou bien un groupe alkyle cyclique ayant 8 à 30 atomes de carbone, tel que isobornyle; ou encore R₂ représente un groupe isobutyle ;

On peut ainsi citer l'acrylate d'éthyle-2-hexyle, le (méth)acrylate d'isobornyle, le (méth)acrylate de lauryle, le (méth)acrylate de stéaryle, le (méth)acrylate de béhényle, l'acrylate d'isobutyle et le méthacrylate de tertiobutyle, et leurs mélanges.

Comme monomère insoluble susceptible d'être employé, on peut citer, seul ou en mélange, les monomères suivants, ainsi que leurs sels et leurs mélanges:
- (i) les (méth)acrylates de formule : CH₂=C(CH₃)-COOR'₁ ou CH₂=CH-COOR'₁ dans laquelle R'1 représente un groupe choisi parmi :
   - un groupe alkyle, linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4; et/ou pouvant être substitué par au moins un groupe polyoxyalkylène, en particulier avec un alkylène en C2-C4, notamment un polyoxyéthylène et/ou un polyoxy-propylène, ledit groupe polyoxyalkylène étant constitué par la répétition de 5 à 30 motifs oxyalkylène; sont exclus de cette définition le méthacrylate de tertiobutyle et l'acrylate d'isobutyle.
   - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, CI, Br, I);
   A titre d'exemples de R'1, on peut citer le groupe méthyle, éthyle, propyle, butyle, méthoxyéthyle, éthoxyéthyle, méthoxy-polyoxyéthylène 30, trifluoroéthyle, 2-hydroxyéthyle, 2-hydroxypropyle, diméthylaminoéthyle, diéthylaminoéthyle, diméthylaminopropyle.
- (ii) l'acide acrylique, l'acide méthacrylique, et leurs sels.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alcanols amines comme la monoéthanolamine, la diéthanolamine, la triéthanolamine, la 2-méthyl-2-amino-1-propanol.
On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Comme monomère insoluble particulièrement préféré, on peut citer :
les (méth)acrylates de méthyle, d'éthyle, de propyle, de butyle; le méthacrylate d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle;
l'acide (méth)acrylique et ses sels.

Plus particulièrement, on peut citer le (méth)acrylate de méthyle, le (méth)acrylate d'éthyle, l'acide (méth)acrylique.

La dispersion de particules de polymères selon l'invention comprend également un milieu carboné liquide selon la présente revendication 1 dans lequel sont dispersées lesdites particules.

Par milieu liquide, on entend notamment un milieu ayant de préférence une viscosité inférieure ou égale à 7000 centipoises à 20°C.

Selon l'invention, le milieu est dit carboné, s'il comprend au moins 50% en poids, notamment de 50 à 100% en poids, par exemple de 60 à 99% en poids, ou encore de 65 à 95% en poids, voire de 70 à 90% en poids, par rapport au poids total du milieu carboné, de composé carboné, liquide à 25°C, ayant un paramètre de solubilité global selon l'espace de solubilité de HANSEN inférieur ou égal à 20 (MPa)^{1/2}, ou d'un mélange de tels composés.

Le paramètre de solubilité global δ selon l'espace de solubilité de HANSEN est défini dans l'article "Solubility parameter values" dans l'ouvrage "Polymer Handbook" 3ème édition, Chapitre VII, pages 519-559, éd. Brandrup, Immergut et Grulke; Wiley Interscience Publication, par la relation : δ = (d_{D}² + d_{P}² + d_{H}²)^{1/2}
dans laquelle :
- d_{D} caractérise les forces de dispersion de LONDON issues de la formation de dipôles induits lors des chocs moléculaires,
- d_{P} caractérise les forces d'interactions de DEBYE entre dipôles permanents,
- d_{H} caractérise les forces d'interactions spécifiques (type liaisons hydrogène, acide/base, donneur/accepteur, etc.).
La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés carbonés liquides ayant un paramètre de solubilité global selon l'espace de solubilité de HANSEN inférieur ou égal à 20 (MPa)^{1/2}, on peut citer les corps gras liquides, notamment les huiles, qui peuvent être choisies parmi les huiles naturelles ou synthétiques, carbonées, hydrocarbonées, éventuellement fluorées, éventuellement ramifiées, seules ou en mélange.

En particulier, on peut citer :
- les huiles végétales formées par des esters d'acides gras et de polyols, en particulier les triglycérides, telles que l'huile de tournesol, de sésame ou de colza, de macadamia, de soja, l'huile d'amande douce, de calophyllum, de palme, de pépins de raisin, de maïs, d'arara, de coton, d'abricot, d'avocat, de jojoba, d'olive ou de germes de céréales;
- les esters linéaires, ramifiés ou cycliques, ayant plus de 6 atomes de carbone, notamment 6 à 30 atomes de carbone; et notamment l'isononanoate d'isononyle ; et plus particulièrement les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle ;
- les hydrocarbures et notamment les alcanes linéaires, ramifiés et/ou cycliques, volatils ou non volatils, tels que les isoparaffines en C₅-C₆₀, éventuellement volatiles tels que l'isododécane, le Parléam (polyisobutène hydrogéné), l'isohexadécane, le cyclohexane, ou les 'ISOPARs'; ou bien les huiles de paraffine, de vaseline, ou le polyisobutylène hydrogéné.

- les éthers ayant plus de 6 atomes de carbone, notamment 6 à 30 atomes de carbone ;
- les cétones ayant plus de 6 atomes de carbone, notamment 6 à 30 atomes de carbone.
- les monoalcools gras aliphatiques ayant 6 à 30 atomes de carbone, la chaîne hydrocarbonée ne comportant pas de groupement de substitution, tels que l'alcool oléique, le décanol, le dodécanol, l'octadécanol, l'octyldodécanol et l'alcool linoléique;
- les polyols notamment ayant 6 à 30 atomes de carbone, tels que l'hexylène glycol;
- leurs mélanges.
la dispersion comprend dans le milieu carboné, au moins un composé carboné choisi parmi :
- les esters de formule RCOOR' dans laquelle R représente le reste d'un acide gras supérieur comportant 7 à 19 atomes de carbone et R' représente une chaîne hydrocarbonée comportant de 3 à 20 atomes de carbone,
- les alcanes linéaires ou ramifiés en C8-C60, volatils ou non volatils;
- les alcanes cycliques, non aromatiques, en C5-C12; volatils ou non volatils;
- les monoalcools gras aliphatiques ayant 12 à 30 atomes de carbone, la chaîne hydrocarbonée ne comportant pas de groupement de substitution,
- leurs mélanges.

Préférentiellement, le milieu carboné comprend au moins, comme composé carboné, du myristate d'isopropyle, de l'octyldodécanol, des isoparaffines en C5-C60, de l'isohexadécane, l'isononanoate d'isononyle.

Le milieu carboné peut éventuellement comprendre des composés liquides additionnels qui peuvent être présents en une quantité strictement inférieure à 50% en poids, notamment de 1 à 40% en poids, voire de 5 à 35% en poids, ou encore de 10 à 30% en poids, par rapport au poids total du milieu carboné, et choisis parmi, seul ou en mélange :
- les huiles siliconées, volatiles ou non volatiles, seules ou en mélange ;
On peut notamment citer les polydiméthylsiloxanes et les polyméthylphénylsiloxa-nes, éventuellement substitués par des groupements aliphatiques et/ou aromatiques, éventuellement fluorés, et/ou comportant des groupements fonctionnels tels que des groupements hydroxyles, thiols et/ou amines; et les siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.
- les esters ayant 2 à 5 atomes de carbone, les éthers ayant 2 à 6 atomes de carbone, les cétones 1 à 5 atomes de carbone, les monoalcools ayant 1 à 5 atomes de carbone.

Toutefois, selon un mode particulier de réalisation de l'invention, le milieu carboné ne contient pas de composés liquides additionnels.

Le choix du milieu carboné peut être effectué aisément par l'homme du métier en fonction de la nature des monomères constituant le polymère et/ou de la destination de la composition.

Parmi les dispersions tout particulièrement préférées, on peut citer les dispersions de particules de poly(acrylate de 2-ethyle hexyle)-b-p(acrylate de méthyle), ou bien de poly(acrylate d'isobornyle)-b-poly(acrylate de méthyle), ou encore de poly(acrylate de 2-ethyle hexyle-co-acrylate d'isobornyle)-b-poly(acrylate de méthyle), et en particulier dans un alcane et notamment dans l'isododécane.

On peut encore citer les dispersions des polymères suivants, notamment dans les alcanes et en particulier dans l'isododécane:
- poly(acrylate de 2-éthyle hexyle)-b-poly(acrylate de méthyle-co-acide acrylique)
- poly(acrylate de 2-ethyle hexyle-co-acide acrylique)-b-poly(acrylate de méthyle)
- poly(acrylate de 2-ethyle hexyle-co-acrylate d'isobornyle)-b-poly(acrylate de méthyle-co-acide acrylique)
- poly(acrylate de 2-ethyle hexyle-co-acrylate d'isobornyle-co-acide acrylique)-b-poly(acrylate de méthyle)
- poly(acrylate d'isobornyle)-b-poly(acrylate de méthyle-co-acide acrylique)
- poly(acrylate d'isobornyle-co-acide acrylique)-b-poly(acrylate de méthyle)
- poly(acrylate d'isobutyle)-b-poly(acrylate de méthyle)
- poly(acrylate d'isobutyle)-b-poly(acrylate de méthyle-co-acide acrylique)
- poly(acrylate d'isobutyle-co-acide acrylique)-b-poly(acrylate de méthyle)
- poly(acrylate d'isobutyle-co-acrylate d'isobornyle)-b-poly(acrylate de méthyle)
- poly(acrylate d'isobutyle-co-acrylate d'isobornyle)-b-poly(acrylate de méthyle-co-acide acrylique)
- poly(acrylate d'isobutyle-co-acrylate d'isobornyle-co-acide acrylique)-b-poly(acrylate de méthyle)
- poly(acrylate de 2-ethyle hexyle)-b-poly(acrylate de méthyle)-b-poly(acrylate de 2-ethyle hexyle)
- poly(acrylate de 2-ethyle hexyle-co-acrylate d'isobornyle)-b-poly(acrylate de méthyle)-b- poly(acrylate de 2-ethyle hexyle-co-acrylate d'isobornyle)
- poly(acrylate de 2-ethyle hexyle-co-acide acrylique)-b-poly(acrylate de méthyle)-b- poly(acrylate de 2-ethyle hexyle-co-acide acrylique)
- poly(acrylate de 2-ethyle hexyle)-b-poly(acrylate de méthyle-co-acide acrylique)-b- poly(acrylate de 2-ethyle hexyle)
- poly(acrylate de 2-ethyle hexyle-co-acrylate d'isobornyle-co-acide acrylique)-b-poly(acrylate de méthyle)-b-poly(acrylate de 2-ethyle hexyle-co-acrylate d'isobornyle-co-acide acrylique)
- poly(acrylate de 2-ethyle hexyle-co-acrylate d'isobornyle)-b-poly(acrylate de méthyle-co-acide acrylique)-b-poly(acrylate de 2-ethyle hexyle-co-acrylate d'isobornyle)

La dispersion selon l'invention présente de préférence un taux de matière sèche compris entre 5 et 80% en poids, notamment 8 à 70% en poids, voire 10 à 60%, ou encore 15 à 50% en poids, et mieux 18 à 25% en poids.

La dispersion de polymère peut être fabriquée par tout moyen connu de l'homme du métier, et notamment par polymérisation radicalaire contrôlée ou par polymérisation vivante, notamment par les techniques dites nitroxydes/alcoxyamines, ATRP, aux organocobalt, RAFT/MADIX, transfert dégénératif, TERP (tellurium), au Selenium, par Iniferter, ou par tout procédé de polymérisation vivante (anionique ou cationique), par métallocène, par ROMP (ring opening metathesis polymerization), par ROP (ring opening polymerization) cationique ou anionique, par GTP (group transfer polymerization), par les dérivés du tétraphenyléthane, par le di-phénylethylène. Les techniques employées pour la formation de chaque séquence peuvent être identiques ou différentes.

Un procédé type peut consister à préparer la première séquence, dite soluble, dans le milieu carboné de la dispersion, par polymérisation du ou des monomères, d'un agent de contrôle et d'un amorceur si nécessaire. Ensuite, le ou les monomères de la séquence dite insoluble sont ajoutés en présence ou non d'amorceur. La température de réaction est de préférence comprise entre -30 et 200°C, de préférence de 0 à 160°C et plus préférentiellement de 40 à 140°C. Des séquences supplémentaires peuvent être polymérisées selon le même procédé. Pour chacune des séquences, le ou les monomères peuvent être ajoutés simultanément, en batch, en semi-continu ou consécutivement. On obtiendra alors des polymères multiblocs.

Si la première séquence, dite soluble, est synthétisée en masse, la séquence dite insoluble peut être ensuite synthétisée en masse ou en solution. Le solvant peut être un solvant carboné tel que défini dans la présente demande, ce qui conduit à l'issue de la synthèse du copolymère à une dispersion directement dans le milieu carboné. Le solvant employé peut également être un solvant commun à toutes les séquences; dans ce cas, l'ajout ultérieur d'un solvant carboné tel que défini ci-dessus dans la présente demande et l'élimination éventuelle du solvant commun conduira à la dispersion voulue dans le milieu carboné.

Si tout le copolymère est synthétisé en masse, l'ajout d'un solvant carboné tel que défini ci-dessus conduira à la dispersion voulue.

Si toutes les séquences sont synthétisées en solution, dans un solvant commun, l'ajout ultérieur d'un solvant carboné tel que défini ci-dessus et l'élimination éventuelle du solvant commun conduira à la dispersion voulue dans les milieux carbonés. Il est également possible à ce stade d'éliminer le solvant commun afin de récupérer le polymère seul et avant de le disperser dans un solvant carboné tel que défini ci-dessus, ce qui conduira à la dispersion voulue.

Enfin, si toutes les séquences sont synthétisées directement dans un solvant carboné tel que défini ci-dessus, la dispersion est obtenue directement, en une seule étape. Ce dernier procédé est celui utilisé de façon préférentielle.

Une fois la dispersion obtenue, il est possible de changer de milieu carboné par élimination / ajout d'un nouveau solvant carboné ou par ajout / élimination éventuelle du premier solvant.
De préférence, la première séquence est préparée par polymérisation radicalaire contrôlée (PRC), la seconde séquence pouvant être également préparée par PRC ou par polymérisation conventionnelle.

Un second mode opératoire préféré consiste à synthétiser la séquence soluble en masse, puis à la solubiliser dans un solvant carboné selon l'invention, puis à synthétiser la séquence insoluble dans ce solvant carboné; on obtient ainsi directement une dispersion du polymère dans le solvant carboné.

Dans un mode de réalisation particulier de l'invention, une fois la dispersion obtenue, il est possible d'y ajouter un ou plusieurs monomères C dont les homopolymères sont soit de type solubles, soit de type insolubles, selon la définition donnée plus haut, et de préférence insolubles, dans le milieu, afin de continuer la polymérisation sur les copolymères à blocs A-B déjà formés, ce qui conduit à la formation de copolymères triblocs A-B-C.
Le ou les monomères additionnels C peuvent être présents en une quantité telle que les quantités de monomères solubles et insolubles totales restent dans les fourchettes totales mentionnées ci-dessus.
Dans le cas où le copolymère de départ est un tribloc de structure A-B-A, la polymérisation de C peut conduire à un pentabloc de structure C-A-B-A-C ou A-B-C-B-A, selon la technique de polymérisation et/ou l'agent de transfert employé.

L'amorceur de polymérisation peut être tout amorceur connu de l'homme de l'art pour la polymérisation radicalaire (peroxydes, azoïques, couple rédox, photochimique). Dans le cas de certaines techniques de polymérisation radicalaire contrôlée, un même composé peut avoir pour rôle d'amorcer la polymérisation et d'être l'agent de contrôle comme c'est le cas des alcoxyamines. Pour les polymérisations non radicalaires, c'est-à-dire ioniques (anionique ou cationique), l'homme de l'art peut choisir l'amorceur adéquat.

On obtient ainsi des copolymères qui s'auto-organisent en dispersion dans le milieu considéré. Ils sont composés d'une première séquence soluble A et d'au moins une seconde séquence, insoluble, B, ce qui va provoquer une auto-organisation des chaînes de polymères de manière à former des particules présentant à l'interface avec le milieu les séquences A et au coeur de la particule les séquences B. Une fois, la dispersion obtenue, il est possible d'y ajouter des dispersants, des stabilisants, pour en modifier les propriétés physicochimiques (viscosité, Tg, etc).

Les dispersions selon l'invention trouvent une application toute particulière en cosmétique. Ainsi, elles peuvent être présentes dans les compositions cosmétiques selon l'invention en une quantité de 0,1 à 90% en poids, de préférence 0,5 à 80% en poids, notamment 1 à 75% en poids, voire 5-70% en poids, de dispersion par rapport au poids total de la composition.

Les compositions cosmétiques selon l'invention comprennent en outre, un milieu cosmétiquement acceptable, c'est-à-dire compatible avec les matières kératiniques telles que la peau du visage ou du corps, les lèvres, les cheveux, les cils, les sourcils et les ongles.

La composition peut avantageusement comprendre une phase grasse, qui peut elle-même comprendre des huiles et/ou des solvants de préférence lipophiles, ainsi que des corps gras solides à température ambiante tels que les cires, les corps gras pâteux, les gommes et leurs mélanges.
Parmi les constituants de la phase grasse, on peut citer les huiles, volatiles ou non, qui peuvent être choisies parmi les huiles naturelles ou synthétiques, carbonées, hydrocarbonées, fluorées, éventuellement ramifiées, seules ou en mélange. On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).
On peut en particulier citer les huiles non volatile carbonées, notamment hydrocarbonées, d'origine végétale, minérale, animale ou synthétique, telles que l'huile de paraffine (ou vaseline), le squalane, le polyisobutène hydrogéné (Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.
On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol.
On peut encore citer les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.
Parmi les composés volatils, on peut citer les huiles volatiles non siliconées, notamment les isoparaffines en C8-C16 comme l'isododécane, l'isodécane, l'isohexadécane. Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.
La phase grasse peut être présente en une teneur allant de 0,01 à 95%, de préférence de 0,1 à 90%, de préférence encore de 10 à 85% en poids, par rapport au poids total de la composition, et mieux de 30 à 80%.

La composition peut également comprendre, une phase hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en C₂ et des aldéhydes en C₂-C₄ hydrophiles. L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes. Par cire au sens de la présente invention, on entend un composé lipophile, solide à température ambiante (25 °C), à changement d'état solide/liquide réversible, ayant un point de fusion supérieur ou égal à 30°C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30 % en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans la composition, en une teneur allant de 0,01 à 50% en poids, par rapport au poids de la composition, de préférence de 0,01 à 30% en poids. Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 s⁻¹, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
La composition selon l'invention peut être une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.
La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

L'invention a aussi pour objet un procédé cosmétique de maquillage, de nettoyage, de protection solaire, de mise en forme, de coloration, ou de soin des matières kératiniques, notamment de la peau du corps ou du visage, des ongles, des cheveux et/ou des cils, comprenant l'application sur lesdites matières d'une composition cosmétique telle que définie précédemment.

L'invention est illustrée plus en détails dans les exemples suivants, donnés à titre d'illustration.

### Exemples 1 à 4

### 1/ Synthèse du bloc soluble

Les différents composants du mélange (monomère, agent de transfert, amorceur) sont mis en présence et homogénéisés par agitation dans un ballon rotaflo® muni d'un septum. Le ballon est alors introduit dans de l'azote liquide et soumis au vide durant une minute une fois le mélange gelé. Le mélange est alors dégelé et libère de l'oxygène résiduel. Ce cycle gel/dégel est répété 5 fois de manière à éliminer l'oxygène du mélange. Le ballon est ensuite introduit dans un bain d'huile thermostaté à 80°C. Il en est retiré et refroidi sous courant d'eau froide après des temps variables de réaction. Les blocs ainsi obtenus, c'est à dire les poly(acrylate de 2-éthylhexyle) fonctionnalisés soit DTB (dithiobenzoate de tertiobutyle), soit TTC (bis(2-éthylhexyl)-2,2'-(trithiocarbonate)dipropanoate), sont précipités deux fois à froid dans le méthanol, puis séchés sous cloche à pression réduite.

| | |
|---|---|
| 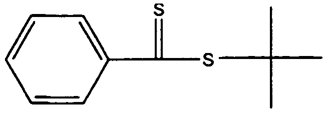 | structure du DTB |
| 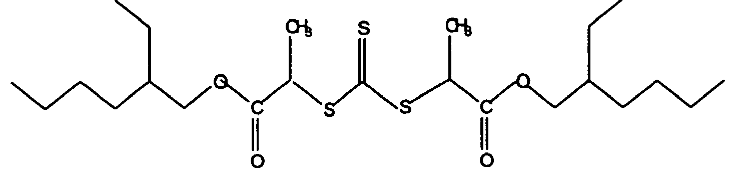 | structure du TTC |

| **Bloc 1a** | Monomère | Agent de transfert | Amorceur | Durée | Taux de conversion | Mn théorique* | Mn exp. (g/mol) / Ip |
|---|---|---|---|---|---|---|---|
| nature | acrylate de 2-éthylhexyle | DTB | T21S | | | | 18200 / 1.14 |
| masse | 61.52 g | 647.3 mg | 222.5 mg | 4h | 82.5% | 16500 | |

| **Bloc 1b** | Monomère | Agent de transfert | Amorceur | Durée | Conversion | Mn théorique* | Mn exp. (g/mol) / Ip |
|---|---|---|---|---|---|---|---|
| nature | Acrylate de 2-éthylhexyle | DTB | T21S | | | | 12600 / 1.14 |
| masse | 15.39 g | 164.6 mg | 57.3 mg | 2h | 58% | 11500 | |

| **Bloc 1c** | Monomère | Agent de transfert | Amorceur | Durée | Conversion | Mn théorique* | Mn exp. (g/mol) / Ip |
|---|---|---|---|---|---|---|---|
| nature | Acrylate de 2-éthylhexyle | TTC | T21S | | | | 21000 / 1.15 |
| masse | 15.38 g | 368.9 mg | 17.1 mg | 4h | 80.2% | 16000 | |

| **Bloc 1d** | Monomère | Agent de transfert | Amorceur | Durée | Conversion | Mn théorique* | Mn exp. (g/mol) / Ip |
|---|---|---|---|---|---|---|---|
| nature | Acrylate de 2-éthylhexyle | TTC | T21S | | | | 15000 / 1.65 |
| masse | 30.77 g | 737.8 mg | 33.1 mg | 4h | 83% | 16500 | |

| | | | | | | | |
|---|---|---|---|---|---|---|---|
| * Mn théorique en g/mol, à la conversion obtenue expérimentalement du bloc soluble ** T21 S : Trigonox 21 S, tert-butyl peroxy-2-ethylhexanoate | | | | | | | |

### 2/ Synthèse du bloc insoluble, formation de dispersions

Les différents composants du mélange (monomère, bloc soluble, amorceur, solvant) sont mis en présence et homogénéisés par agitation dans un ballon rotaflo® muni d'un septum. Le ballon est alors introduit dans de l'azote liquide et soumis au vide durant une minute une fois le mélange gelé. Le mélange est alors dégelé et libère de l'oxygène résiduel. Ce cycle gel/dégel est répété 5 fois de manière à éliminer l'oxygène du mélange. Le ballon est ensuite introduit dans un bain d'huile thermostaté à 80°C. Il en est retiré et refroidi sous courant d'eau froide après des temps variables de réaction. Une dispersion de copolymères à blocs est ainsi obtenue.

Les exemples 1 et 2 concernent des copolymères diblocs. Les exemples 3 et 4 concernent des copolymères triblocs de type soluble-insoluble-soluble.

| **Ex. 1** | Bloc soluble | Amorceur | Monomère | Solvant | Durée / conversion | Mn théorique** | Mn exp.*** (g/mol) / Ip. |
|---|---|---|---|---|---|---|---|
| nature | Bloc 1a | T21S | Acrylate de Méthyle | Isododecane 10g | | | 28000 / 6 |
| masse | 1.5 g | 105.6 mg* | 3.05 g | | 24 h / 70% | 27000 | |

| **Ex. 2** | Bloc soluble | Amorceur | Monomère | Solvant | Durée / conversion | Mn théorique** | Mn exp.*** (g/mol) / Ip. |
|---|---|---|---|---|---|---|---|
| nature | Bloc 1b | T21S | Acrylate de Méthyle | Isododecane | | | 168000 / 4.25 |
| masse | 1.5 g | 106.2 mg* | 4.47g | 15g | 24 h / 69% | 26400 | |

| **Ex. 3** | Bloc soluble | Amorceur | Monomère | Solvant | Durée / conversion | Mn théorique** | Mn exp.*** (g/mol) / Ip. |
|---|---|---|---|---|---|---|---|
| nature | Bloc 1c | T21S | Acrylate de Méthyle | Isododecane | | | 28500 / 1.34 |
| masse | 1.5 g | 109.3 mg* | 2.62 g | 8.6 g | 2 h / 70% | 25900 | |

| **Ex. 4** | Bloc soluble | Amorceur | Monomère | Solvant | Durée / conversion | Mn théorique** | Mn exp.*** (g/mol) / Ip. |
|---|---|---|---|---|---|---|---|
| nature | Bloc 1d | T21S | Acrylate de Méthyle | Isododecane | | | 25100 / 3.33 |
| masse | 1g | 102.9 mg* | 2.42 g | 8.2 g | 4 h / 62% | 23600 | |

| | | | | | | | |
|---|---|---|---|---|---|---|---|
| * ex1: solution de T21 S à 2.6 10⁻¹ mol/L dans l'acrylate de méthyle * ex2: solution de T21 S à 3.8 10⁻¹ mol/L dans l'acrylate de méthyle * ex3: solution de T21 S à 2.3 10⁻¹ mol/L dans l'acrylate de méthyle * ex4: solution de T21 S à 2.1 10⁻¹ mol/L dans l'acrylate de méthyle ** : Mn théorique du bloc insoluble en g/mol, à la conversion obtenue expérimentalement *** : Mn expérimentale du bloc insoluble en g/mol, Ip du copolymère à bloc | | | | | | | |

### Exemple 5

L'acrylate de 2-éthylhexyle (4 g), le DTB (dithiobenzoate de tertiobutyle, 52,5 mg) et le Trigonox 21S (18 mg) sont mis en présence et homogénéisés par agitation dans un ballon rotaflo® muni d'un septum. Le ballon est alors introduit dans de l'azote liquide et soumis au vide durant une minute une fois le mélange gelé. Le mélange est alors dégelé et libère de l'oxygène résiduel. Ce cycle gel/dégel est répété 5 fois de manière à éliminer l'oxygène du mélange. Le ballon est ensuite introduit dans un bain d'huile thermostaté à 80°C. Après 6 heures de réaction, on effectue un prélèvement à la seringue (bloc 1e), puis on introduit à la canule un mélange acrylate de méthyle (6,72 g) / T21S (17,5 mg) / isododécane (21,45 g), préalablement dégazé par bullage d'azote (trente minutes). Le ballon est laissé 22 heures supplémentaires dans le bain à 80°C. Le ballon est alors refroidi sous courant d'eau froide. On obtient une dispersion de particules de polymères dans l'isododécane.

| | Mn théorique | Mn exp*** (g/mol) | Ip |
|---|---|---|---|
| Bloc1e | 17 000* | 17 000 | 1,13 |
| Dispersion | 25 300** | 55600 | 6 |

| | | | |
|---|---|---|---|
| * : Mn théorique en g/mol du bloc soluble, à conversion totale ** : Mn théorique en g/mol du bloc insoluble, à conversion totale *** : Mn expérimentales en g/mol pour chacun des blocs | | | |

### Exemple 6 : caractérisations des dispersions des exemples 1 à 5

| Exemple | Extrait sec | diamètre des particules (PDI) | % massique de fraction soluble | % molaire de fraction soluble |
|---|---|---|---|---|
| 1 | 28% | 50 nm | 39.4% | 23,2% |
| | | (0,22) | | |
| 2 | 24% | 80 nm | 7% | 3,4% |
| | | (0,10) | | |
| 3 | 30% | 35 nm | 42.4% | 25,7% |
| | | (0,09) | | |
| 4 | 24% | 50 nm | 37.4% | 21,9% |
| | | ( 0,07) | | |
| 5 | 28% | 40 nm | 23,1% | 12,2% |

| | | | | |
|---|---|---|---|---|
| PDI : indice de polydispersité qui traduit la polydispersité en taille de particules | | | | |

Les conversions sont mesurées par RMN ¹H, pour les blocs solubles, et par gravimétrie pour les blocs insolubles.
Les masses molaires par GPC dans le THF avec des étalons de polystyrènes linéaires.
Les diamètres moyens des particules sont mesurés par diffusion dynamique de la lumière avec un Malvern Nano-S90 en tenant compte de l'indice de réfraction et de la viscosité du solvant.

### Exemple 7 : Composition de mascara

On a préparé un mascara ayant la composition suivante :

| | |
|---|---|
| Cires | 17 g |
| Hectorite modifiée (Bentone^{®} 38V d'Elementis) | 5,3 g |
| Carbonate de propylène | 1,7 g |
| Charge | 1g |
| pigments | 5 g |
| Dispersion de polymère de l'exemple 1 | 10 g MS* |
| Isododécane | qsp 100 g |

| | |
|---|---|
| *MS : matière sèche | |

Le mascara, après application sur les cils, est jugé très satisfaisant.

### Exemple 8 : Stick de rouge à lèvres

La composition de rouge à lèvres suivante est préparée :

| | |
|---|---|
| Cire | 15 % |
| Dispersion de polymère de l'exemple 2 | 10 % en MS |
| Huile carbonée non volatile | 26 % |
| Pigments | 8.6 % |
| Isododécane | qsp 100% |

La composition obtenue après application sur les lèvres présente de bonnes propriétés cosmétiques.

### Exemple 9 : Fond de teint E/H

On prépare une composition de fond de teint comprenant les composés suivants :

| Phase A | |
|---|---|
| Cetyl Dimethicone copolyol | 3 g |
| (ABIL EM 90 de la société GOLDSCHMIDT) | |
| Succinate d'isostéaryl diglycéryle | 0,6 g |
| (IMWITOR 780K de la société CONDEA) | |
| Isododécane | 18,5 g |
| Pigments (oxydes de fer et oxydes de titane hydrophobes) | 10 g |
| Dispersion de polymère de l'exemple 3 | 8 g en MS |
| Charge | 8 g |
| Parfum | qs |
| | |

| Phase B | |
|---|---|
| Eau | qsp 100 g |
| Sulfate de magnésium | 0,7 g |
| Conservateur (Methylparaben) | qs |
| | |

| Phase C | |
|---|---|
| Eau | 2 g |
| Conservateur (Diazolinyl urée) | qs |

La composition obtenue présente de bonnes propriétés cosmétiques.

### Exemple 10 : Poudre compactée

On prépare une poudre compactée ayant la composition suivante :

| *Composition A :* | |
|---|---|
| - Talc | 30 g |
| - Oxychlorure de bismuth | 10 g |
| - Stéarate de zinc | 4 g |
| - Poudre de Nylon | 20 g |
| - Dispersion de l'exemple 4 | 5 g |
| | |

| *Composition B :* | |
|---|---|
| - Oxydes de fer | 2 g |
| - Huile de vaseline | 6 g |

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant de bonnes propriétés cosmétiques. La composition obtenue est aisée et agréable à appliquer. On constate que le film ne migre pas dans les ridules de la peau, même après avoir été porté pendant plusieurs heures.

### Exemple 11 : gel pour le visage

On prépare la composition suivante :

| | |
|---|---|
| . isopropyl palmitate | 10 g |
| . vaseline (cire) | 5 g |
| . hectorite modifiée (argile) | 0,15 g |
| . ozokérite (cire) | 5 g |
| . septaoléate de sorbitane oxyéthyléné (400E) | 5 g |
| . dispersion de l'exemple 5 | 75 g |

On obtient un gel ayant de bonnes propriétés cosmétiques.

### Exemple 12 : huile de soin

On prépare la composition suivante :

| | |
|---|---|
| . dispersion de l'exemple 2 | 70 g |
| . huile de jojoba | 15 g |
| . huile de soja | 15 g |

On obtient une huile de soin qui peut être appliquée sur le corps ou le visage.

## Revendications

1. Composition cosmétique comprenant, dans un milieu cosmétiquement acceptable, au moins une dispersion de particules de polymères dans un milieu carboné liquide, ledit polymère étant un copolymère comprenant au moins une première séquence soluble dans ledit milieu carboné et au moins une deuxième séquence insoluble dans ledit milieu carboné;
ladite séquence soluble comprenant 50 à 100% en poids de monomère(s) soluble(s) dans ledit milieu, seul ou en mélange, et 0 à 50% en poids de monomère(s) insoluble(s) dans ledit milieu, seul ou en mélange;
ladite séquence insoluble comprenant 50 à 100% en poids de monomère(s) insoluble(s) dans ledit milieu, seul ou en mélange; et 0 à 50% en poids de monomère(s) soluble(s) dans ledit milieu, seul ou en mélange;
ledit copolymère comprenant au moins un monomère soluble choisi parmi, seul ou en mélange, les monomères suivants :
- les méthacrylates de formule CH₂ = C(CH₃)-COOR₁ dans laquelle R₁ représente un groupe alkyle linéaire ou ramifié en C8-C22, ou un groupe alkyle cyclique ayant 8 à 30 atomes de carbone, ou R₁ représente le groupe tertiobutyle;
- les acrylates de formule CH₂ = CH-COOR₂ dans laquelle R₂ représente un groupe alkyle linéaire ou ramifié en C8-C22 ou un groupe alkyle cyclique ayant 8 à 30 atomes de carbone; ou R₂ représente un groupe isobutyle;
ledit copolymère comprenant au moins un monomère insoluble choisi parmi, seul ou en mélange, les monomères suivants, ainsi que leurs sels, :
- les (méth)acrylates de formule CH₂=C(CH₃)-COOR'₁ ou CH₂=CH-COOR'₁ dans laquelle R'1 représente un groupe alkyle, linéaire ou ramifié, comprenant 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène et -NR'R" avec R' et R", identiques ou différents, choisis parmi les alkyles, linéaires ou ramifiés, en C1-C4; sont exclus de cette définition le méthacrylate de tertiobutyle et l'acrylate d'isobutyle;
- l'acide acrylique, l'acide méthacrylique, et leurs sels;
ladite dispersion comprenant dans le milieu carboné, au moins un composé carboné choisi parmi :
- les esters de formule RCOOR' dans laquelle R représente le reste d'un acide gras supérieur comportant 7 à 19 atomes de carbone et R' représente une chaîne hydrocarbonée comportant de 3 à 20 atomes de carbone,
- les alcanes linéaires ou ramifiés en C8-C60, volatils ou non volatils;
- les alcanes cycliques, non aromatiques, en C5-C12; volatils ou non volatils;
- les monoalcools gras aliphatiques ayant 12 à 30 atomes de carbone, la chaîne hydrocarbonée ne comportant pas de groupement de substitution,
- leurs mélanges.

2. Composition selon la revendication 1, dans laquelle le copolymère présente un indice de polydispersité en masse (Ip) inférieur ou égal à 6 de préférence compris entre 1,05 et 4, notamment entre 1,1 et 3, voire entre 1,15 et 2,5.

3. Composition selon l'une des revendications précédentes, dans laquelle chaque séquence est de type homopolymére ou à gradient.

4. Composition selon l'une des revendications précédentes, dans laquelle le copolymère est du type 'dibloc', 'tribloc' ou 'multi-blocs'; de préférence dibloc.

5. Composition selon l'une des revendications précédentes, dans laquelle le copolymère est linéaire.

6. Composition selon l'une des revendications précédentes, dans laquelle les particules sont d'une taille comprise entre 5 et 1000 nm, de préférence 10 à 500 nm, encore mieux 20 à 300 nm, voire de 30 à 200 nm.

7. Composition selon l'une des revendications précédentes, dans laquelle le copolymère a un poids moléculaire moyen en nombre (Mn) compris entre 1000 à 700 000, notamment entre 10 000 et 500 000, et encore mieux entre 15 000 et 350 000, voire entre 25 000 et 150 000.

8. Composition selon l'une des revendications précédentes, dans laquelle la séquence soluble comprend 60 à 90% en poids de monomère(s) soluble(s) dans ledit milieu, notamment 70 à 80% en poids de monomère(s) soluble(s), seul ou en mélange.

9. Composition selon l'une des revendications précédentes, dans laquelle la séquence insoluble comprend 60 à 90% en poids de monomère(s) insoluble(s) dans ledit milieu, notamment de 70 à 80% en poids de monomére(s) insoluble(s), seul ou en mélange.

10. Composition selon l'une des revendications précédentes, dans laquelle la séquence insoluble (ou les séquences insolubles), représente 30 à 97% en poids du poids total du copolymère, notamment de 40 à 95% en poids, voire de 50 à 93% en poids, mieux de 60 à 92% en poids, et encore de 75 à 90% en poids; et la séquence soluble (ou les séquences solubles), représente 3 à 70% en poids du poids total du copolymère, notamment de 5 à 60% en poids, voire de 7 à 50% en poids, mieux 8 à 40% en poids, et encore 10 à 25% en poids.

11. Composition selon l'une des revendications précédentes, dans laquelle le monomère soluble est choisi parmi l'acrylate d'éthyle-2-hexyle, le (méth)acrylate d'isobornyle, le (méth)acrylate de lauryle, le (méth)acrylate de stéaryle, le (méth)acrylate de béhényle, l'acrylate d'isobutyle et le méthacrylate de tertiobutyle, et leurs mélanges.

12. Composition selon l'une des revendications précédentes, dans laquelle le monomère insoluble est choisi parmi le (méth)acrylate de méthyle, le (méth)acrylate d'éthyle, l'acide (méth)acrylique.

13. Composition selon l'une des revendications précédentes, dans laquelle le milieu carboné comprend au moins 50% en poids, notamment de 50 à 100% en poids, par exemple de 60 à 99% en poids, ou encore de 65 à 95% en poids, voire de 70 à 90% en poids, par rapport au poids total du milieu carboné, de composé carboné, liquide à 25°C, ayant un paramètre de solubilité global selon l'espace de solubilité de HANSEN inférieur ou égal à 20 (MPa)^{1/2}, ou d'un mélange de tels composés.

14. Composition selon l'une des revendications précédentes, dans laquelle le composé carboné est choisi parmi, seul ou en mélange, le myristate d'isopropyle, l'octyldodécanol, les isoparaffines en C5-C60, l'isohexadécane, l'isononanoate d'isononyle.

15. Composition selon l'une des revendications précédentes, dans laquelle la dispersion est une dispersion de particules :
- de poly(acrylate de 2-ethyle hexyle)-b-poly(acrylate de méthyle),
- de poly(acrylate d'isobornyle)-b-poly(acrylate de méthyle),
- de poly(acrylate de 2-ethyle hexyle-co-acrylate d'isobornyle)-b-poly(acrylate de méthyle),
- poly(acrylate de 2-éthyle hexyle)-b-poly(acrylate de méthyle-co-acide acrylique)
- poly(acrylate de 2-ethyle hexyle-co-acide acrylique)-b-poly(acrylate de méthyle)
- poly(acrylate de 2-ethyle hexyle-co-acrylate d'isobornyle)-b-poly(acrytate de méthyle-co-acide acrylique)
- poly(acrylate de 2-ethyle hexyle-co-acrylate d'isobornyle-co-acide acrylique)-b-poly(acrylate de méthyle)
- poly(acrylate d'isobornyle)-b-poly(acrylate de méthyle-co-acide acrylique)
- poly(acrylate d'isobornyle-co-acide acrylique)-b-poly(acrylate de méthyle)
- poly(acrylate d'isobutyle)-b-poly(acrylate de méthyle)
- poly(acrylate d'isobutyle)-b-poly(acrylate de méthyle-co-acide acrylique)
- poly(acrylate d'isobutyle-co-acide acrylique)-b-poly(acrylate de méthyle)
- poly(acrylate d'isobutyle-co-acrylate d'isobornyle)-b-poly(acrylate de méthyle)
- poly(acrylate d'isobutyle-co-acrylate d'isobornyle)-b-poly(acrylate de méthyle-co-acide acrylique)
- poly(acrylate d'isobutyle-co-acrylate d'isobornyle-co-acide acrylique)-b-poly(acrylate de méthyle)
- poly(acrylate de 2-ethyle hexyle)-b-poly(acrylate de méthyle)-b-poly(acrylate de 2-ethyle hexyle)
- poly(acrylate de 2-ethyle hexyle-co-acrylate d'isobornyle)-b-poly(acrylate de méthyle)-b- poly(acrylate de 2-ethyle hexyle-co-acrylate d'isobornyle)
- poly(acrylate de 2-ethyle hexyle-co-acide acrylique)-b-poly(acrylate de méthyle)-b- poly(acrylate de 2-ethyle hexyle-co-acide acrylique)
- poly(acrylate de 2-ethyle hexyle)-b-poly(acrylate de méthyle-co-acide acrylique)-b- poly(acrylate de 2-ethyle hexyle)
- poly(acrylate de 2-ethyle hexyle-co-acrylate d'isobornyle-co-acide acrylique)-b-poly(acrylate de méthyle)-b-poly(acrylate de 2-ethyle hexyle-co-acrylate d'isobornyle-co-acide acrylique)
- poly(acrylate de 2-ethyle hexyle-co-acrylate d'isobornyle)-b-poly(acrylate de méthyle-co-acide acrylique)-b-poly(acrylate de 2-ethyle hexyle-co-acrylate d'isobornyle)

16. Composition selon la revendication 15, dans laquelle la dispersion est dans un alcane et en particulier dans l'isododécane.

17. Composition selon l'une des revendications précédentes, dans laquelle la dispersion présente un taux de matière sèche compris entre 5 et 80% en poids, notamment 8 à 70% en poids, voire 10 à 60% en poids, ou encore 15 à 50% en poids, mieux 18 à 25% en poids.

18. Composition selon l'une des revendications précédentes, dans laquelle la dispersion est présente en une quantité de 0,1 à 90% en poids, de préférence 0,5 à 80% en poids, notamment 1 à 75% en poids, voire 5-70% en poids, de dispersion par rapport au poids total de la composition.

19. Composition selon l'une des revendications précédentes, comprenant en outre, au moins un constituant choisi parmi les phases grasses, les phases hydrophiles, les matières colorantes, les polymères, les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les céramides, ou leurs mélanges.

20. Composition selon l'une des revendications précédentes, se présentant sous forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel; d'un produit capillaire, notamment pour le maintien de la coiffure ou la mise en forme des cheveux.

21. Procédé cosmétique de maquillage, de nettoyage, de protection solaire, de mise en forme, de coloration, ou de soin des matières kératiniques, notamment de la peau du corps ou du visage, des ongles, des cheveux et/ou des cils, comprenant l'application sur lesdites matières d'une composition cosmétique selon l'une des revendications 1 à 20.

## Claims

1. Cosmetic composition comprising, in a cosmetically acceptable medium, at least one dispersion of polymer particles in a liquid carbon-based medium, said polymer being a copolymer comprising at least one first block that is soluble in said carbon-based medium and at least one second block that is insoluble in said carbon-based medium;
said soluble block comprising 50 to 100% by weight of monomer(s) that is (are) soluble in said medium, alone or as a mixture, and 0 to 50% by weight of monomer(s) that is (are) insoluble in said medium, alone or as a mixture;
said insoluble block comprising 50 to 100% by weight of monomer(s) that is (are) insoluble in said medium, alone or as a mixture; and 0 to 50% by weight of monomer(s) that is (are) soluble in said medium, alone or as a mixture;
said copolymer comprising at least one soluble monomer chosen from, alone or as a mixture, the following monomers:
- the methacrylates of formula CH₂=C(CH₃)-COOR₁ in which R₁ represents a linear or branched C₈-C₂₂ alkyl group or a cyclic alkyl group containing 8 to 30 carbon atoms, or R₁ represents the tert-butyl group;
- the acrylates of formula CH₂=CH-COOR₂ in which R₂ represents a linear or branched C₈-C₂₂ alkyl group or a cyclic alkyl group containing 8 to 30 carbon atoms; or R₂ represents an isobutyl group;
said copolymer comprising at least one insoluble monomer chosen from, alone or as a mixture, the following monomers, and also salts thereof:
- the (meth)acrylates of formula CH₂=C(CH₃)-COOR'₁ or CH₂=CH-COOR_{'1} in which R'₁ represents a linear or branched alkyl group containing 1 to 6 carbon atoms, it being possible for said group to comprise, in its chain, one or more heteroatoms chosen from O, N and S; and/or to comprise one or more substituents chosen from -OH, halogen atoms and -NR'R" with R' and R", which may be identical or different, chosen from linear or branched C₁-C₄ alkyls; with tert-butyl methacrylate and isobutyl acrylate being excluded from this definition;
- acrylic acid and methacrylic acid, and salts thereof;
said dispersion comprising, in the carbon-based medium, at least one carbon-based compound chosen from:
- the esters of formula RCOOR' in which R represents the residue of a higher fatty acid containing 7 to 19 carbon atoms and R' represents a hydrocarbon-based chain containing from 3 to 20 carbon atoms;
- volatile or non-volatile, linear or branched C₈-C₆₀ alkanes;
- volatile or non-volatile, non-aromatic cyclic C₅-C₁₂ alkanes;
- aliphatic fatty monoalcohols containing 12 to 30 carbon atoms, the hydrocarbon-based chain not comprising a substitution group;
- mixtures thereof.

2. Composition according to Claim 1, in which the copolymer has a weight polydispersity index (PI) of less than or equal to 6, preferably between 1.05 and 4, in particular between 1.1 and 3, or even between 1.15 and 2.5.

3. Composition according to either of the preceding claims, in which each block is of homopolymer or gradient type.

4. Composition according to one of the preceding claims, in which the copolymer is of the "diblock", "triblock" or "multiblock" type, preferably diblock type.

5. Composition according to one of the preceding claims, in which the copolymer is linear.

6. Composition according to one of the preceding claims, in which the particles are of a size between 5 and 1000 nm, preferably from 10 to 500 nm, even better still from 20 to 300 nm, or even from 30 to 200 nm.

7. Composition according to one of the preceding claims, in which the copolymer has a number-average molecular weight (Mn) of between 1000 and 700 000, in particular between 10 000 and 500 000, and even better still between 15 000 and 350 000, or even between 25 000 and 150 000.

8. Composition according to one of the preceding claims, in which the soluble block comprises from 60 to 90% by weight of monomer(s) that is (are) soluble in said medium, in particular from 70 to 80% by weight of soluble monomer(s), alone or as a mixture.

9. Composition according to one of the preceding claims, in which the insoluble block comprises from 60 to 90% by weight of the monomer(s) that is (are) insoluble in said medium, in particular from 70 to 80% by weight of insoluble monomer(s), alone or as a mixture.

10. Composition according to one of the preceding claims, in which the insoluble block (or the insoluble blocks) represents (or represent) from 30 to 97% by weight of the total weight of the copolymer, in particular from 40 to 95% by weight, or even from 50 to 93% by weight, better still from 60 to 92% by weight, and a further 75 to 90% by weight, and the soluble block (or the soluble blocks) represents (or represent) from 3 to 70% by weight of the total weight of the copolymer, in particular from 5 to 60% by weight, or even from 7 to 50% by weight, better still from 8 to 40% by weight, and further 10 to 25% by weight.

11. Composition according to one of the preceding claims, in which the soluble monomer is chosen from 2-ethylhexyl acrylate, isobornyl (meth)acrylate, lauryl (meth)acrylate, stearyl (meth)acrylate, behenyl (meth)acrylate, isobutyl acrylate and tert-butyl methacrylate, and mixtures thereof.

12. Composition according to one of the preceding claims, in which the insoluble monomer is chosen from methyl (meth)acrylate, ethyl (meth)acrylate and (meth)acrylic acid.

13. Composition according to one of the preceding claims, in which the carbon-based medium comprises at least 50% by weight, in particular from 50 to 100% by weight, for example from 60 to 99% by weight, or further 65 to 95% by weight, or even from 70 to 90% by weight, relative to the total weight of the carbon-based medium, of carbon-based medium that is liquid at 25°C, with an overall solubility parameter according to the Hansen solubility space of less than or equal to 20 (MPa)^{1/2}, or of a mixture of such compounds.

14. Composition according to one of the preceding claims, in which the carbon-based compound is chosen from, alone or as a mixture, isopropyl myristate, octyldodecanol, C₅-C₆₀ isoparaffins, isohexadecane and isononyl isononanoate.

15. Composition according to one of the preceding claims, in which the dispersion is a dispersion of particles of:
- poly(2-ethylhexyl acrylate)-b-poly(methyl acrylate),
- poly(isobornyl acrylate)-b-poly(methyl acrylate),
- poly(2-ethylhexyl acrylate-co-isobornyl acrylate)-b-poly(methyl acrylate),
- poly(2-ethylhexyl acrylate)-b-poly(methyl acrylate-co-acrylic acid),
- poly(2-ethylhexyl acrylate-co-acrylic acid)-b-poly(methyl acrylate),
- poly(2-ethylhexyl acrylate-co-isobornyl acrylate)-b-poly(methyl acrylate-co-acrylic acid),
- poly(2-ethylhexyl acrylate-co-isobornyl acrylate-co-acrylic acid)-b-poly(methyl acrylate),
- poly(isobornyl acrylate)-b-poly(methyl acrylate-co-acrylic acid),
- poly(isobornyl acrylate-co-acrylic acid)-b-poly(methyl acrylate),
- poly(isobutyl acrylate)-b-poly(methyl acrylate),
- poly(isobutyl acrylate)-b-poly(methyl acrylate-co-acrylic acid),
- poly(isobutyl acrylate-co-acrylic acid)-b-poly(methyl acrylate),
- poly(isobutyl acrylate-co-isobornyl acrylate)-b-poly(methyl acrylate),
- poly(isobutyl acrylate-co-isobornyl acrylate)-b poly(methyl acrylate-co-acrylic acid),
- poly(isobutyl acrylate-co-isobornyl acrylate-co-acrylic acid)-b-poly(methyl acrylate),
- poly(2-ethylhexyl acrylate)-b-poly(methyl acrylate)-b-poly(2-ethylhexyl acrylate),
- poly(2-ethylhexyl acrylate-co-isobornyl acrylate)-b-poly(methyl acrylate)-b-poly(2-ethylhexyl acrylate-co-isobornyl acrylate),
- poly(2-ethylhexyl acrylate-co-acrylic acid)-b-poly(methyl acrylate)-b-poly(2-ethylhexyl acrylate-co-acrylic acid),
- poly(2-ethylhexyl acrylate)-b-poly(methyl acrylate-co-acrylic acid)-b-poly(2-ethylhexyl acrylate),
- poly(2-ethylhexyl acrylate-co-isobornyl acrylate-co-acrylic acid)-b-poly(methyl acrylate)-b-poly(2-ethylhexyl acrylate-co-isobornyl acrylate-co-acrylic acid) ,
- poly(2-ethylhexyl acrylate-co-isobornyl acrylate)-b-poly(methyl acrylate-co-acrylic acid)-b-poly(2-ethylhexyl acrylate-co-isobornyl acrylate).

16. Composition according to Claim 15, in which the dispersion is in an alkane and in particular in isododecane.

17. Composition according to one of the preceding claims, in which the dispersion has a dry matter content of between 5 and 80% by weight, in particular from 8 to 70% by weight, or even from 10 to 60% by weight, or further 15 to 50% by weight, better still from 18 to 25% by weight.

18. Composition according to one of the preceding claims, in which the dispersion is present in an amount of from 0.1 to 90% by weight, preferably from 0.5 to 80% by weight, in particular from 1 to 75% by weight, or even 5-70% by weight, of dispersion relative to the total weight of the composition.

19. Composition according to one of the preceding claims, also comprising at least one constituent chosen from fatty phases, hydrophilic phases, colorants, polymers, vitamins, thickeners, gelling agents, trace elements, demulcents, sequestering agents, fragrances, basifying or acidifying agents, preservatives, sunscreens, surfactants, antioxidants, anti-hairloss agents, anti-dandruff agents, propellants, and ceramides, or mixtures thereof.

20. Composition according to one of the preceding claims, which is in the form of a makeup composition, in particular a complexion product such as a foundation, a face powder or an eye shadow; a lip product such as a lipstick or a lipcare product; a concealer product; a blusher, a mascara, an eyeliner; an eyebrow makeup product, a lip pencil or an eye pencil; a nail product such as a nail varnish or a nailcare product; a body makeup product; a hair makeup product (hair mascara or hair lacquer); a composition for protecting or caring for the skin of the face, the neck, the hands or the body, in particular an anti-wrinkle composition, an anti-fatigue composition for giving the skin radiance, a moisturizing or treating composition; an antisun or self-tanning composition; a hair product, in particular for retaining the hairstyle or for shaping the hair.

21. Cosmetic process for making up, cleansing, sun-protecting, shaping, dyeing or caring for keratin materials, in particular bodily or facial skin, the nails, the hair and/or the eyelashes, comprising the application to said materials of a cosmetic composition according to one of Claims 1 to 20.

## Patentansprüche

1. Kosmetikzusammensetzung, die in einem kosmetisch annehmbaren Medium mindestens eine Dispersion von Polymerpartikeln in einem flüssigen Medium auf Kohlenstoffbasis umfasst, wobei es sich bei dem Polymer um ein Copolymer handelt, das mindestens eine erste Sequenz, die in dem Medium auf Kohlenstoffbasis löslich ist, und mindestens eine zweite Sequenz, die in dem Medium auf Kohlenstoffbasis unlöslich ist, umfasst;
wobei die lösliche Sequenz 50 bis 100 Gew.-% Monomer(e), das/die in dem Medium löslich ist/sind, allein oder in Abmischung, und 0 bis 50 Gew.-% Monomer(e), das/die in dem Medium unlöslich ist/sind, allein oder in Abmischung, umfasst;
wobei die unlösliche Sequenz 50 bis 100 Gew.-% Monomer(e), das/die in dem Medium unlöslich ist/sind, allein oder in Abmischung, und 0 bis 50 Gew.-% Monomer(e), das/die in dem Medium löslich ist/sind, allein oder in Abmischung, umfasst;
wobei das Copolymer mindestens ein lösliches Monomer umfasst, das, allein oder in Abmischung, aus den folgenden Monomeren ausgewählt ist:
- Methacrylaten der Formel CH₂=C(CH₃)-COOR₁, worin R₁ eine geradkettige oder verzweigte C₈-C₂₂-Alkylgruppe oder eine cyclische Alkylgruppe mit 8 bis 30 Kohlenstoffatomen bedeutet, oder R₁ die tert.-Butylgruppe bedeutet;
- Acrylaten der Formel CH₂=CH-COOR₂, worin R₂ eine geradkettige oder verzweigte C₈-C₂₂-Alkylgruppe oder eine cyclische Alkylgruppe mit 8 bis 30 Kohlenstoffatomen bedeutet, oder R₂ eine Isobutylgruppe bedeutet;
wobei das Copolymer mindestens ein unlösliches Monomer umfasst, das, allein oder in Abmischung, aus den folgenden Monomeren ausgewählt ist, sowie ihren Salzen:
- (Meth)acrylaten der Formel CH₂=C(CH₃)COOR'₁ oder CH₂=CH-COOR'₁, worin R'₁ eine geradkettige oder verzweigte Alkylgruppe mit 1 bis 6 Kohlenstoffatome bedeutet, wobei die Gruppe in ihrer Kette ein oder mehrere Heteroatome aus der Reihe O, N und S umfassen kann; und/oder einen oder mehrere Substituenten aus der Reihe -OH, Halogenatome und -NR'R", wobei R' und R", die gleich oder verschieden sind, aus der Reihe geradkettige oder verzweigte C₁-C₄-Alkylreste ausgewählt sind, umfassen kann; wobei tert.-Butylmethacrylat und Isobutylacrylat aus dieser Definition ausgenommen sind;
- Acrylsäure, Methacrylsäure und ihren Salzen;
wobei die Dispersion in dem Medium auf Kohlenstoffbasis mindestens eine Verbindung auf Kohlenstoffbasis umfasst, die aus der folgenden Reihe ausgewählt ist:
- Ester der Formel RCOOR', worin R einen höheren Fettsäurerest mit 7 bis 19 Kohlenstoffatomen bedeutet und R' eine Kohlenwasserstoffkette mit 3 bis 20 Kohlenstoffatomen bedeutet,
- flüchtige oder nichtflüchtige geradkettige oder verzweigte C₈-C₆₀-Alkane;
- flüchtige oder nichtflüchtige cyclische nichtaromatische C₅-C₁₂-Alkane;
- aliphatische fette Monoalkohole mit 12 bis 30 Kohlenstoffatomen, wobei die Kohlenwasserstoffkette keine Substitutionsgruppe aufweist,
- ihre Mischungen.

2. Zusammensetzung nach Anspruch 1, wobei das Copolymer einen Masse-Polydispersitätsindex (Ip) von 6 oder darunter, vorzugsweise zwischen 1,05 und 4, insbesondere zwischen 1,1 und 3, sogar zwischen 1,15 und 2,5, aufweist.

3. Zusammensetzung nach einem der vorhergehenden Ansprüche, wobei jede Sequenz den Homopolymertyp oder den Gradiententyp aufweist.

4. Zusammensetzung nach einem der vorhergehenden Ansprüche, wobei das Copolymer den ,Diblock'-, den ,Triblock'- oder den ,Multi-Block'-Typ, vorzugsweise den Diblock-Typ aufweist.

5. Zusammensetzung nach einem der vorhergehenden Ansprüche, wobei das Copolymer linear ist.

6. Zusammensetzung nach einem der vorhergehenden Ansprüche, wobei die Partikel eine Größe zwischen 5 und 1000 nm, vorzugsweise 10 bis 500 nm, stärker bevorzugt 20 bis 300 nm, sogar 30 bis 200 nm, aufweisen.

7. Zusammensetzung nach einem der vorhergehenden Ansprüche, wobei das Copolymer ein zahlenmittleres Molekulargewicht (Mn) zwischen 1000 bis 700000, insbesondere zwischen 10000 und 500000, stärker bevorzugt zwischen 15000 und 350000, sogar zwischen 25000 und 150000, aufweist.

8. Zusammensetzung nach einem der vorhergehenden Ansprüche, wobei die lösliche Sequenz 60 bis 90 Gew.-% Monomer(e), das/die in dem Medium löslich ist/sind, insbesondere 70 bis 80 Gew.-% lösliche(s) Monomer(e), allein oder in Abmischung, umfasst.

9. Zusammensetzung nach einem der vorhergehenden Ansprüche, wobei die unlösliche Sequenz 60 bis 90 Gew.- % Monomer(e), das/die in dem Medium unlöslich ist/sind, insbesondere 70 bis 80 Gew.-% unlösliche(s) Monomer(e), allein oder in Abmischung, umfasst.

10. Zusammensetzung nach einem der vorhergehenden Ansprüche, wobei die unlösliche(n) Sequenz(en) 30 bis 97 Gew.-% des Gesamtgewichts des Copolymers, insbesondere 40 bis 95 Gew.-%, sogar 50 bis 93 Gew.-%, bevorzugt 60 bis 92 Gew.-%, stärker bevorzugt 75 bis 90 Gew.-% ausmacht und die lösliche(n) Sequenz(en) 3 bis 70 Gew.-% des Gesamtgewichts des Copolymers, insbesondere 5 bis 60 Gew.-%, sogar 7 bis 50 Gew.-%, stärker bevorzugt 8 bis 40 Gew.-%, noch stärker bevorzugt 10 bis 25 Gew.-% ausmacht.

11. Zusammensetzung nach einem der vorhergehenden Ansprüche, wobei das lösliche Monomer aus der Reihe 2-Hexylethylacrylat, Isobornyl(meth)acrylat, Lauryl (meth)acrylat, Stearyl(meth)acrylat, Behenyl (meth)acrylat, Isobutylacrylat und tert.-Butyl methacrylat und ihren Mischungen ausgewählt ist.

12. Zusammensetzung nach einem der vorhergehenden Ansprüche, wobei das unlösliche Monomer aus der Reihe Methyl(meth)acrylat, Ethyl(meth)acrylat, (Meth)acrylsäure ausgewählt ist.

13. Zusammensetzung nach einem der vorhergehenden Ansprüche, wobei das Medium auf Kohlenstoffbasis mindestens 50 Gew.-%, insbesondere 50 bis 100 Gew.-%, zum Beispiel 60 bis 99 Gew.-% oder auch 65 bis 95 Gew.- %, sogar 70 bis 90 Gew.-%, in Bezug auf das Gesamtgewicht des Mediums auf Kohlenstoffbasis an einer bei 25°C flüssigen Verbindung auf Kohlenstoffbasis mit einem Gesamtsolubilitätsparameter gemäß dem Löslichkeitsraum nach HANSEN von 20 (Mpa)^{1/2} oder darunter, oder eine Mischung von solchen Verbindungen aufweist.

14. Zusammensetzung nach einem der vorhergehenden Ansprüche, wobei die Verbindung auf Kohlenstoffbasis aus der Reihe Isopropylmyristat, Octyldodecanol, C₅-C₆₀-Isoparaffine, Isohexadecan, Isononylisononanoat, allein oder in Abmischung, ausgewählt ist.

15. Zusammensetzung nach einem der vorhergehenden Ansprüche, wobei es sich bei der Dispersion um eine Dispersion aus den folgenden Partikeln handelt:
- Poly(2-ethylhexylacrylat)-b-poly(methylacrylat),
- Poly(isobornylacrylat)-b-poly(methylacrylat),
- Poly(2-ethylhexylacrylat-co-isobornylacrylat)-b-poly(methylacrylat),
- Poly(2-ethylhexylacrylat)-b-poly(methylacrylat-co-acrylsäure),
- Poly(2-ethylhexylacrylat-co-acrylsäure)-b-poly(methylacrylat),
- Poly(2-ethylhexylacrylat-co-isobornylacrylat)-b-poly(methylacrylat-co-acrylsäure),
- Poly(2-ethylhexylacrylat-co-isobornylacrylat-co-acrylsäure)-b-poly(methylacrylat),
- Poly(isobornylacrylat)-b-poly(methylacrylat-co-acrylsäure),
- Poly(isobornylacrylat-co-acrylsäure)-b-poly(methylacrylat),
- Poly(isobutylacrylat)-b-poly(methylacrylat),
- Poly(isobutylacrylat)-b-poly(methylacrylat-co-acrylsäure),
- Poly(isobutylacrylat-co-acrylsäure)-b-poly(methylacrylat),
- Poly(isobutylacrylat-co-isobornylacrylat)-b-poly(methylacrylat),
- Poly(isobutylacrylat-co-isobornylacrylat)-b-poly(methylacrylat-co-acrylsäure),
- Poly(isobutylacrylat-co-isobornylacrylat-co-acrylsäure)-b-poly(methylacrylat),
- Poly(2-ethylhexylacrylat)-b-poly(methylacrylat)-b-poly(2-ethylhexylacrylat),
- Poly(2-ethylhexylacrylat-co-isobornylacrylat)-b-poly(methylacrylat)-b-poly(2-ethylhexylacrylat-co-isobornylacrylat),
- Poly(2-ethylhexylacrylat-co-acrylsäure)-b-poly(methylacrylat)-b-poly(2-ethylhexylacrylat-co-acrylsäure),
- Poly(2-ethylhexylacrylat)-b-poly(methylacrylat-co-acrylsäure)-b-poly(2-ethylhexylacrylat),
- Poly(2-ethylhexylacrylat-co-isobornylacrylat-co-acrylsäure)-b-poly(methylacrylat)-b-poly(2-ethylhexylacrylat-co-isobornylacrylat-co-acrylsäure),
- Poly(2-ethylhexylacrylat-co-isobornylacrylat)-b-poly(methylacrylat-co-acrylsäure)-b-poly(2-ethylhexylacrylat-co-isobornylacrylat).

16. Zusammensetzung nach Anspruch 15, wobei die Dispersion in einem Alkan und insbesondere in Isododecan vorliegt.

17. Zusammensetzung nach einem der vorhergehenden Ansprüche, in der die Dispersion in einem Trockenmasseanteil zwischen 5 und 80 Gew.-%, insbesondere 8 und 70 Ges.-%, sogar 10 und 60 Gew.-% oder auch 15 und 50 Gew.-%, stärker bevorzugt 18 und 25 Gew.-% vorliegt.

18. Zusammensetzung nach einem der vorhergehenden Ansprüche, in der die Dispersion in einer Menge von 0,1 bis 90 Ges.-%, vorzugsweise 0,5 bis 80 Gew.-%, insbesondere 1 bis 75 Gew.-%, sogar 5 bis 70 Gew.-%, Dispersion im Vergleich zu dem Gesamtgewicht der Zusammensetzung vorliegt.

19. Zusammensetzung nach einem der vorhergehenden Ansprüche, die weiterhin mindestens einen Bestandteil, ausgewählt aus der Reihe Fettphasen, hydrophile Phasen, Farbmittel, Polymere, Vitamine, Verdickungsmittel, Geliermittel, Spurenelemente, zartmachende Mittel, Sequestriermittel, Parfums, Alkalinisierungs- oder Säuerungsmittel, Konservierungsmittel, Sonnenschutzfilter, Tenside, Antioxidantien, Mittel gegen Haarausfall, Mittel gegen Schuppen, Treibstoffe, Ceramide oder ihre Mischungen umfasst.

20. Zusammensetzung nach einem der vorhergehenden Ansprüche, die in Form einer Make-up-Zusammensetzung vorliegt, insbesondere ein Produkt für den Teint, wie eine Make-up-Grundlage, ein Wangenrouge oder ein Lidschatten; ein Produkt für die Lippen, wie ein Lippenfärbe- oder- Pflegeprodukt; ein Produkt gegen Falten; ein Rouge, ein Mascara, ein Eyeliner; ein Augenbrauenschminkprodukt, ein Lippenstift oder ein Lidschattenstift; ein Produkt für die Nägel wie ein Nagellack oder ein Nagelpflegemittel; ein Körperschminkprodukt; ein Haarschminkprodukt (Haarmascara oder -lack); eine Zusammensetzung für den Schutz oder die Pflege der Gesichtshaut, des Halses, der Hände oder des Körpers, insbesondere eine Zusammensetzung gegen Falten, gegen die Müdigkeit, welches der Haut ein frisches Aussehen verleiht, eine feuchtigkeitsspendende oder pflegende Zusammensetzung; ein Sonnenschutzmittel oder ein künstliches Bräunungsmittel; ein Haarprodukt, wie für die Festigung der Frisur oder zum Formen des Haares.

21. Kosmetikprodukt für das Schminken, die Reinigung, den Sonnenschutz, das Formen, das Färben und die Pflege von Keratinsubstanzen, insbesondere der Haut des Körpers oder des Gesichts, der Nägel, des Haares und/oder der Wimpern, bei dem man eine Kosmetikzusammensetzung nach einem der Ansprüche 1 bis 20 auf diese Substanzen aufträgt.
